# FASCICULE DE BREVET EUROPEEN

(11) **EP 3 288 627 B1**
(45) Date de publication et mention de la délivrance du brevet: **29.05.2019**
(21) Numéro de dépôt: 16726177.5
(22) Date de dépôt: 29.04.2016
(51) Int. Cl.: A61M 16/04, A61M 25/02

(54) **DISPOSITIF DE PROTECTION ET DE MAINTIEN D'UNE SONDE DESTINEE A METTRE L'INTERIEUR DU CORPS D'UN PATIENT EN COMMUNICATION AVEC L'EXTERIEUR**
VORRICHTUNG ZUM SCHÜTZEN UND HALTEN EINER SONDE ZUR PLATZIERUNG INNERHALB DES KÖRPERS EINES PATIENTEN IN KOMMUNIKATION MIT DER AUSSENSEITE
DEVICE FOR PROTECTING AND HOLDING IN POSITION A PROBE THAT IS INTENDED TO BE PLACED INSIDE THE BODY OF A PATIENT IN COMMUNICATION WITH THE OUTSIDE

(30) Priorité: 30.04.2015 FR 1553948
(43) Date de publication de la demande: 07.03.2018
(73) Titulaire: MMG, 34600 Les Aires (FR); SORBONNE UNIVERSITE, 75006 Paris (FR)
(72) Inventeur: SIMILOWSKI, Thomas, 92130 Issy-les-moulineaux (FR); RENAUX, Serge, 34600 Les Aires (FR)
(74) Mandataire: Gevers & Orès
(86) Numéro de dépôt international: PCT/IB2016/052451
(87) Numéro de publication internationale: WO 2016/174632

(56) Documents cités:
- DE-U1-202006 001 328
- US-A- 4 270 529
- US-A1- 2014 332 009

## Description

L'invention se situe dans le domaine des dispositifs d'aide à la mise en place et au maintien d'une sonde, destiné à mettre l'intérieur du corps d'un patient en communication avec l'extérieur.

Dans le domaine médical, il est courant d'avoir à mettre en communication l'intérieur du corps d'un patient avec un dispositif adapté aux soins à apporter, par exemple un système d'aspiration ou un système de ventilation artificielle.

La ventilation artificielle à l'aide d'une sonde est la mesure d'assistance vitale la plus fréquemment employée en situation d'urgence et en réanimation. Elle peut être administrée au moyen de différentes « interfaces » entre le patient et le « ventilateur ». L'intubation trachéale constitue l'une de ces interfaces, préférée dans les situations les plus graves, ou lorsque l'état du patient requiert une sédation. Elle consiste à introduire une sonde dans les voies aériennes : l'extrémité distale de la sonde est placée dans la trachée, son extrémité proximale est reliée au « ventilateur ». Bien que l'intubation trachéale puisse être réalisée par voie nasale ou par voie buccale, c'est la voie buccale qui est actuellement quasi-exclusivement utilisée en pratique.

Une fois la sonde mise en place, il est nécessaire de la fixer et de la protéger d'éventuelles morsures par le patient. Ces deux objectifs sont fondamentaux, pour la qualité de l'assistance ventilatoire : un sonde qui se déplace peut rendre celle-ci inefficace ou dangereuse (déplacement vers l'extérieur ou « extubation » : absence de ventilation : déplacement vers l'intérieur : risque d'intubation dite « sélective » conduisant à ne plus ventiler qu'un seul poumon et non les deux). Une sonde mordue ou pliée peut rendre la ventilation inefficace voire impossible.

Un dispositif de ce type est connu par exemple de US-A-4,270,529, se rapportant à un dispositif permettant de stabiliser la position d'une sonde endotrachéale, et permettant de protéger celle-ci d'éventuels dommages par morsure à l'aide d'un bloc de plastique. Un autre dispositif de ce type est connu de US 2014/0332009, concernant un bloc de morsure configuré pour englober un tube endotrachéal, et pour protéger la bouche d'un patient entubé lors d'un mouvement de serrement de la mâchoire.

La fixation des sondes d'intubation oro-trachéales peut-être réalisée par différent moyens :
- On peut utiliser pour cela un simple cordonnet lacé sur la sonde, éventuellement protégé par une gaine plastique ; le cordonnet n'assure pas une fixation parfaite de la sonde, et pas de protection ; de plus, devant être serré autour de la tête du patient, il peut induire inconfort et lésions (escarres aux points de friction, comme la commissure des lèvres ou les oreilles) ;
- On peut ajouter au cordonnet une canule endobuccale dite « de Guedel » qui ajoute à l'inconfort de la présence de la sonde dans la bouche.

Il convient de noter qu'au-delà de la nécessité de fixer et protéger la sonde d'intubation, les soins aux malades ventilés impliquent parfois la nécessité de la mobiliser, pour en optimiser la position après un déplacement accidentel quel que soit son origine (spontané, détecté par un examen de contrôle systématique ; accidentel, par le patient ou le personnel soignant à l'occasion d'un déplacement, d'un transport ou d'un soin ; procédural, à l'occasion par exemple d'une endoscopie bronchique). Par ailleurs, la prévention des infections nosocomiales et le confort du patient passent par la réalisation pluriquotidienne, chez les patients ventilés et intubés par la bouche, de soins de bouche. La qualité de ces soins impose un accès aisé à l'ensemble de la cavité buccale et donc, parfois, de libérer la sonde. Devoir retirer et remettre en place un dispositif de fixation de sonde d'intubation représente donc un risque, et du temps de travail. Les dispositifs actuels, notamment un cordonnet, ne rendent pas cette tâche aisée. Il faut rappeler que les deux extrémités de la sonde sont engagées : l'extrémité distale dans la trachée du patient (où un ballonnet à basse pression gonflable de l'extérieur par l'intermédiaire d'un petit raccord assure l'étanchéité), l'extrémité proximale étant reliée au « ventilateur ». Dès lors un dispositif de maintien et de protection de la sonde doit pouvoir se fixer sur la sonde et s'enlever sans passer par une extrémité (et donc, en particulier, sans avoir à déconnecter la sonde du ventilateur) Avec les dispositifs de l'état de l'art, cette opération nécessite l'usage des deux mains, voire de deux personnes.

La présente invention a pour objet de fournir un dispositif qui puisse se fixer et se détacher d'une seule main d'une sonde dont les deux extrémités sont engagées, dont l'une se trouve à l'intérieur du corps du patient, et qui assure un maintien et une protection efficace de cette sonde. Il importe en effet d'éviter tout mouvement de cette sonde, qui pourrait provoquer des dommages corporels graves au patient.

A cet effet l'invention propose un dispositif de maintien d'une sonde de mise en communication, de l'intérieur du corps d'un patient avec l'extérieur, caractérisé en ce qu'il comprend, disposés le long d'un axe longitudinal du dispositif :
- à son extrémité distale, un moyen d'appui du dispositif sur le corps du patient qui s'étend radialement, comportant une ouverture axiale pour le passage de ladite sonde et comportant une première ouverture radiale communiquant avec ladite ouverture axiale,
- à son extrémité proximale, un moyen de guidage de ladite sonde comportant une seconde ouverture radiale,
- un moyen d'écartement longitudinal dudit moyen d'appui et dudit moyen de guidage.

Dans tout le texte, on appellera élément « distal » un élément. par exemple une extrémité, une face... orienté vers le patient en utilisation et élément « proximal » un élément orienté à l'opposé du patient en utilisation.

Pour la mise en place du dispositif sur la sonde. on fait pénétrer celle-ci dans les ouvertures radiales ménagées à la fois dans le moyen d'appui et dans le moyen de guidage ; la sonde prend alors une position le long de l'axe X-X' du dispositif, sans qu'il ait été nécessaire de mobiliser l'une ou l'autre des extrémités de la sonde. Le dispositif se détache de la sonde par la manoeuvre inverse.

La sonde est par exemple une sonde oro-trachéale ou toute autre sonde de mise en communication de l'intérieur du corps d'un patient avec l'extérieur, leur caractéristique commune étant que les deux extrémités de cette sonde ne sont pas mobilisables, c'est à dire que de dispositif ne peut pas être enfilé sur la sonde. La largeur des ouvertures permet le passage de sondes d'une grande variété de diamètres, en jouant sur la plasticité des sondes généralement utilisées.

Le moyen d'appui est destiné à venir au contact des lèvres ou de la peau du patient, son extension radiale dépend de l'application. Il prend par exemple la forme d'une corolle pouvant comporter des éléments de fixation, par exemple des ouvertures, pour un cordonnet destiné à fixer le dispositif sur le corps du patient.

La fonction du moyen de guidage est de maintenir la sonde le long de l'axe longitudinal du dispositif au moins sur la longueur du dispositif. Il présente avantageusement une forme d'anneau coaxial, de préférence torique sans que ceci soit limitatif.

La fonction du moyen d'écartement est simplement de tenir écartés le moyen d'appui et le moyen de guidage. Il prend par exemple la forme d'une plaque, rectiligne ou courbe, dont la longueur dépend également de l'application.

L'invention propose un mode de réalisation en ce qui concerne les ouvertures radiales :
- Dans ce mode de réalisation, la première ouverture radiale et la seconde ouverture radiée sont sensiblement diamétralement opposées par rapport à l'axe longitudinal du dispositif ; cette variante est préférée car elle évite une désolidarisation accidentelle de la sonde et du dispositif,

Le dispositif peut en outre présenter l'une ou l'autre des caractéristiques additionnelles suivantes, seules ou en combinaison :
Dans une application à la fixation d'une sonde oro-trachéale, le moyen d'appui peut être prolongé sur sa face distale par un corps cylindrique coaxial composant une fente longitudinale, ladite fente étant en communication avec l'ouverture radiale du moyen d'appui.

Ce corps cylindrique protège la sonde de la morsure par le patient lorsque le dispositif est mis en place. Sa fente longitudinale en communication avec la première ouverture radiale disposée sur le moyen d'appui et sensiblement de même extension, permet de mettre en place le dispositif sur la sonde et de l'enlever facilement.

Avantageusement, le moyen de guidage peut comporter une excroissance qui s'étend radialement en direction opposée à la pièce d'écartement.

Avantageusement le moyen de guidage peut comporter une languette qui s'étend radialement à partir de l'excroissance.

Le dispositif peut comporter en outre un moyen de blocage prévu pour bloquer la sonde contre le moyen d'écartement lorsque ladite sonde s'étend selon l'axe longitudinal du dispositif

Ce moyen de blocage par frottement évite un glissement relatif de la sonde et du dispositif et évite donc ainsi que la sonde s'échappe du corps du patient ou qu'elle pénètre trop profondément.

Le moyen de blocage peut comprendre une lame qui s'étend longitudinalement à partir de la face proximale du moyen d'appui, diamétralement opposée au moyen d'écartement.

Cette lame est suffisamment mince pour être déformée de façon résiliente, si bien que son extrémité proximale peut se rapprocher ou s'éloigner de l'axe longitudinal du dispositif.

Avantageusement la lame peut présenter une forme radialement incurvée et comporter une partie de blocage qui s'étend vers l'axe longitudinal du dispositif, de façon à bloquer la sonde entre ladite partie de blocage et le moyen d'écartement lorsqu'on exerce une force sur ladite lame.

Cette lame n'est pas rectiligne mais présente une forme de vague, un « creux de vague » étant plus proche de l'axe longitudinal du dispositif La lame et la plaque du moyen d'écartement forment une pince élastique qui permet de bloquer la sonde et d'éviter qu'elle glisse à l'intérieur du dispositif sans écraser la sonde. L'objectif n'est pas en effet, d'interrompre la circulation de gaz ou de liquide dans la sonde mais seulement de la bloquer par frottement. A cet effet, la partie de la plaque qui se trouve en regard de la lame est lisse, ne présente pas de surépaisseur ou de bosse qui viendrait contribuer à cet écrasement.

Avantageusement la face interne du moyen d'écartement peut être munie d'une couche anti-dérapante.

Avantageusement le dispositif peut comporter en outre des moyens d'anti-retour du moyen de blocage dans la position où la partie de blocage est rapprochée du moyen d'écartement et donc dans laquelle la sonde est bloquée dans le dispositif.

Dans cette position, la sonde qui passe selon l'axe longitudinal du dispositif est bloquée contre le moyen d'écartement. Le moyen d'anti-retour maintient la lame et sa partie de blocage contre la sonde. Le moyen d'écartement est plus rigide que la lame, de sorte que lorsqu'on presse la lame contre la sonde et la sonde contre le moyen d'écartement, c'est la lame qui se déforme.

Avantageusement les moyens d'anti-retour peuvent comporter une surface crantée sur une face distale de l'excroissance, lesdits crans étant prévus pour coopérer avec une dent située à l'extrémité proximale du moyen de blocage.

L'extension longitudinale de la lame est sensiblement égale à celle du moyen d'écarternent, si bien que son extrémité proximale peut coopérer avec une série de crans prévus sur une face distale du moyen de guidage. L'extrémité de la lame est libérée des crans en exerçant une force sur la languette tendant à éloigner cette languette du moyen d'appui,

Avantageusement le dispositif peut être monobloc.

Le dispositif est alors composé d'une seule pièce, il ne nécessite aucun assemblage de pièces différentes qui coulissent l'une par rapport à l'autre, ou se vissent l'une par rapport à l'autre.

Le dispositif peut être réalisé en une matière plastique à usage pharmaceutique ou médico-chirurgical.

Ces matières plastiques possèdent intrinsèquement une certaine plasticité, une plus grande rigidité d'un élément étant obtenue lorsqu'on augmente sa compacité, par exemple son épaisseur. C'est ainsi que la plaque qui forme le moyen d'écartement possède une plus grande extension transversale que la lame du moyen de blocage, de façon à être plus rigide que la lame. De la même façon, lorsque le dispositif comporte un corps cylindrique destiné à protéger la sonde de la morsure, l'épaisseur de la paroi du cylindrique est suffisante pour résister à cet effort de morsure.

Avantageusement le moyen de blocage peut comporter un plot disposé sur le moyen d'appui et s'étendant le long de l'axe longitudinal du dispositif à partir de la face proximale dudit moyen d'appui, diamétralement opposée à la face interne de la pièce d'écartement.

Avantageusement le moyen de blocage peut comporter en outre un fermoir présentant la forme d'un prisme droit et comprend dans une partie centrale de son volume une tranchée formant deux bras longitudinaux agencés pour coopérer avec au moins une face du plot.

Avantageusement le fermoir peut comporter des moyens d'anti-retour, lesdits moyens d'anti-retour comprenant des crans disposés l'un en face de l'autre dans la tranchée dudit fermoir et agencés pour s'accrocher à un bord saillant formé à la périphérie du plot.

Avantageusement le fermoir peut comporter un prisme droit à base triangulaire.

Avantageusement au moins l'une des faces du plot et/ou du fermoir est traitée de façon à posséder une propriété antidérapante.

L'invention porte également sur un procédé de mise en place d'un dispositif de maintien d'une sonde selon l'une des revendications précédentes, ladite sonde étant déjà en place sur le corps du patient, comportant les étapes consistant à :
- Introduire la sonde entre l'extrémité proximale du moyen de blocage et le moyen de guidage,
- Faire pénétrer la sonde dans la première ouverture radiale du moyen d'appui (respectivement, dans la seconde ouverture radiale du moyen de guidage),
- Faire pénétrer la sonde dans la seconde ouverture radiale du moyen guidage (respectivement, devant la première ouverture radiale du moyen d'appui),
de façon à ce que la sonde s'étende selon l'axe longitudinal du dispositif.

Ce procédé peut en outre comporter les étapes suivantes :
- Actionner le moyen de blocage.
- Fixer de façon non invasive le dispositif sur le corps du patient.

Ce procède est avantageusement exécuté d'une seule main.

Dans une application à la fixation d'une sonde oro-trachéale, les avantages du dispositif selon l'invention sont les suivants :
- Il protège la sonde oro-trachéale de toute morsure,
- Il occupe dans la bouche du patient et à l'extérieur de celle-ci un espace minimal,
- Il est facile d'usage, permettant une mise en place et un retrait d'une seule main (une main sécurise la sonde, l'autre main mobilise le dispositif de fixation),
- Il peut s'adapter en une seule déclinaison à tous les diamètres de sonde existants. On peut simplement prévoir un modèle adulte et un modèle pédiatrique.

Des modes de réalisation et des variantes seront décrits ci-après, à titre d'exemples non limitatifs, avec référence aux dessins annexés dans lesquels :
Les figures 1 et 3 représentent le dispositif en perspective,
La figure 2 représente le dispositif en élévation,
La figure 4 représente une variante du dispositif en perspective.
La figure 5 représente une variante du dispositif en perspective.

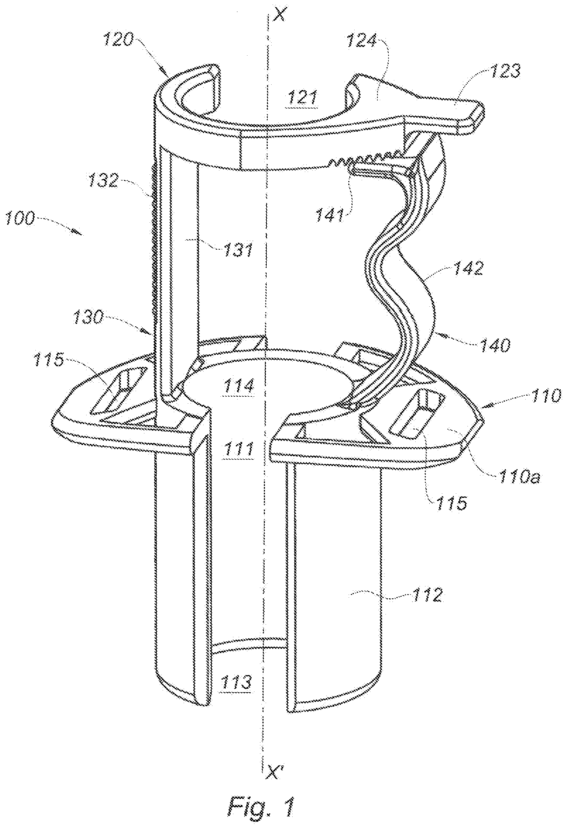

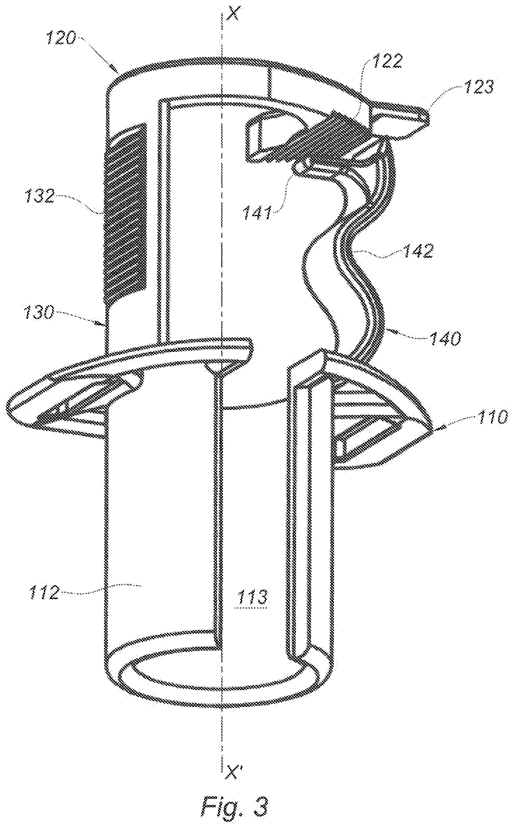

Le dispositif 100 illustré en figures 1 à 4 se développe autour d'un axe rectiligne X-X'. Il est destiné à maintenir en place et à protéger une conduite mettant l'intérieur du corps d'un patient en communication avec l'extérieur, par exemple une sonde oro-trachéale, sans que ceci soit limitatif. Lorsque la sonde est engagée dans le dispositif, elle prend place selon l'axe X-X'.

Le dispositif étant destiné à être appliqué sur le corps d'un patient, il comporte une pièce d'appui 110 qui s'étend radialement autour de l'axe X-X' ; tel qu'illustré, la pièce d'appui 110 prend la forme d'une corolle sensiblement ovale, possédant une face distale 110b destinée à venir en contact avec la peau ou les lèvres du patient et une face proximale 110a à l'opposé. La pièce d'appui 110 est légèrement bombée, la concavité étant tournée vers la face distale 110b c'est-à-dire vers le patient en utilisation, La pièce d'appui 110 comporte une ouverture axiale 114, par exemple circulaire, prévue pour recevoir la sonde.

Cette pièce d'appui 110 comporte en périphérie des ouvertures 115 prévues pour la fixation du dispositif sur le corps du patient, par exemple grâce à un cordonnet.

Le dispositif comporte à son extrémité proximale un anneau 120 qui s'étend, radialement autour de l'axe X-X', formant moyen de guidage pour la sonde et éviter qu'elle ne se plie ou se coude en direction de la face proximale 110a de la pièce d'appui 110 lorsqu'elle est en place. Le plan de la pièce d'appui 110 et de l'anneau 120 sont donc sensiblement parallèles. L'anneau 120 comporte une excroissance 124 qui s'étend radialement en direction opposée à la pièce d'écartement 130, dont la face distale 122 est crantée. L'excroissance 124 se prolonge à l'opposé de l'axe X-X' par une languette 123.

La pièce d'appui 110 et l'anneau 120 sont solidarisés et maintenus à distance par une pièce d'écartement 130 qui s'étend longitudinalement à partir de la face proximale 110a de la pièce d'appui 110, en bordure de son ouverture axiale 114. Cette pièce d'écartement 130 prend la forme d'une plaque allongée, rectiligne longitudinalement et ayant une section transversale incurvée.

Une pièce de blocage 140 s'étend également depuis la face proximale 110a de la pièce d'appui 110 en direction de l'anneau 120, diamétralement opposée par rapport à la pièce d'écartement 130. Son extension longitudinale est sensiblement égale à celle de la pièce d'écartement 130 ; son extrémité proximale est Elle est destinée à bloquer la sonde à l'intérieur du dispositif 100 et sera décrite en détail plus loin.

Le dispositif 100 comporte un corps cylindrique 112 qui s'étend longitudinalement et de façon coaxiale à partir de la face distale 110b de la pièce d'appui 110. Lorsque la sonde est en place dans le dispositif, elle prend place axialement dans ce corps cylindrique 112. Le dispositif de ces figures est destiné notamment à la fixation d'une sonde oro-trachéale et la fonction de ce corps cylindrique est de protéger la sonde des mouvements de morsure involontaires du patient.

Le dispositif 100 comporte des moyens spécifiques pour permettre une mise en place facilitée sur une sonde sont les deux extrémités sont engagées, l'extrémité distale l'intérieur du corps du patient et l'extrémité proximale étant reliée à un appareil. A cet effet, le dispositif comporte ;
- Une première ouverture radiale 111 dans la pièce d'appui 110, communiquant avec l'ouverture axiale 114 de ladite pièce d'appui 110,
- Une seconde ouverture radiale 121 dans l'anneau 120, si bien que l'anneau 120 est interrompu, cette seconde ouverture radiale 121 étant diamétralement opposée par rapport à la première ouverture radiale 111.

Lorsque le dispositif comporte un corps cylindrique 112, celui-ci comporte une fente longitudinale 113 en communication avec la première ouverture 111.

La fixation du dispositif se réalise de la façon suivante :
- Forcer le passage de la sonde entre l'extrémité proximale de la lame 140 et l'excroissance 124 en jouant sur l'élasticité de l'anneau 120 et de la pièce d'écartement ; à la suite de cette manipulation, l'axe de la sonde se trouve sensiblement orthogonal à l'axe X-X',
- Faire pivoter la partie distale de la sonde de façon à l'introduire dans la première ouverture radiale 111, et dans la fente longitudinale 113 lorsque le dispositif comporte un corps cylindrique 112, de façon à la placer dans l'ouverture axiale 114 de la pièce d'appui,
- Faire pivoter la partie proximale de la sonde de façon à l'introduire la sonde dans la seconde ouverture radiale 121, de façon à l'aligner le long de l'axe X-X' du dispositif.

Bien entendu, les deux dernières opérations peuvent être réalisées dans l'ordre inverse.

Il est important de noter que ces opérations peuvent être réalisées d'une seule main par le soignant, ce qui est un avantage important par rapport aux dispositifs de l'état de l'art.

Une fois que le dispositif est en place sur la sonde, il peut librement coulisser sur la sonde sans risque de tomber et peut être mis par le soignant à l'emplacement prévu sur le corps du patient. Dès lors, il convient d'éviter un déplacement longitudinal de l'un par rapport à l'autre. A cet effet, le dispositif comporte des moyens de blocage de la sonde à l'intérieur du dispositif, sous la forme notamment de la pièce de blocage 140.

Telle d'illustrée sur les figures, la pièce de blocage est une lame 140 qui présente une forme ondulée, comportant notamment une « partie creuse » 142, c'est-à-dire plus rapprochée de l'axe X-X' du dispositif Cette « partie creuse » 142 est destinée à fournir un appui pour le doigt de l'opérateur lorsqu'il presse la lame 140.

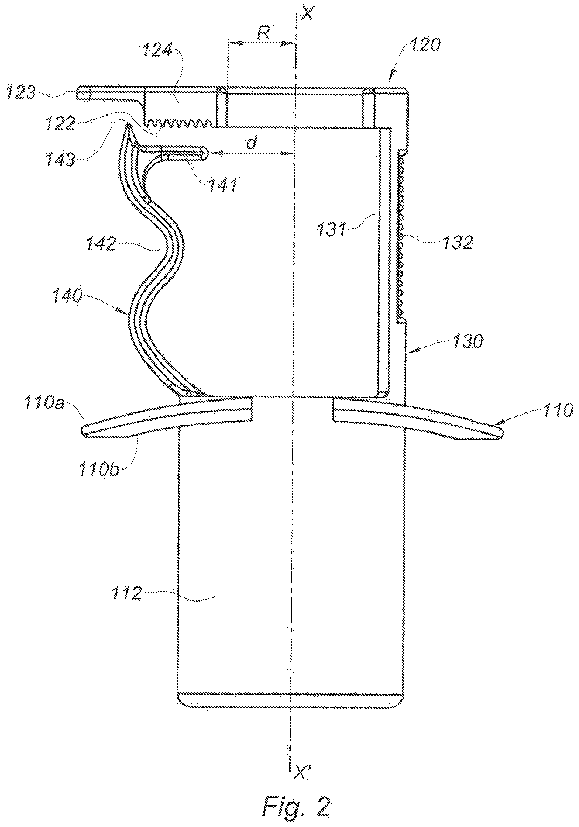

A son extrémité proximale, la pièce de blocage 140 comporte une excroissance 141 formant partie de blocage qui s'étend vers l'axe X-X' du dispositif, prenant la forme d'un bec. Cette excroissance 141 est prévue pour venir en contact avec la sonde pour la bloquer à l'intérieur du dispositif Dans la position d'équilibre de la pièce de blocage 140, telle qu'illustrée en figure 2 par exemple, la distance d entre l'extrémité du bec et l'axe X-X' est supérieure au rayon R de l'anneau 120, de telle sorte qu'elle n'exerce aucune force sur la sonde lorsqu'elle présente dans le dispositif.

Compte tenu de sa position diamétralement opposée par rapport à la pièce d'écartement 130, on comprend que lorsque l'opérateur appuie sur la lame 140, la sonde se trouve bloquée par frottement entre l'excroissance 141 et la face interne 131 de la pièce d'écartement 130.

En pratique, l'opérateur, avec deux doigts d'une même main, appuie sur la face externe de la pièce d'écartement 130 et la face externe de la pièce de blocage 140, à la façon d'une pince. Pour faciliter cette opération, comme il a été vu plus haut, la lace externe de la pièce de blocage 140 comporte une « partie creuse » 142 ; en outre la face externe de la pièce d'écartement 130 comporte une série de rainures 132 prévues pour éviter que le doigt glisse.

De préférence, la pièce d'écartement 130 est plus rigide que la pièce de blocage 140, par exemple parce qu'elle présente une section et/ou une largeur supérieures à celle de la pièce de blocage 140, De cette façon, lors du pinçage du dispositif, c'est préférentiellement la pièce de blocage 140 qui fléchit.

Pour assurer un meilleur blocage de la sonde, la face interne 131 de la pièce d'écartement 130 peut être conformée ou traitée de façon à posséder une propriété antidérapante.

Le dispositif comporte en outre des moyens d'anti-retour des moyens de blocage. A cet effet l'extrémité proximale de la pièce de blocage 140 comporte une dent 143 prévue pour coopérer avec les crans de la face distale 122 de façon, à former un moyen d'anti-retour; les crans sont par exemple asymétriques.

Lorsque la dent 143 est engagée dans les crans de la face distale 122 à la suite d'une pression exercée par l'utilisateur, la pièce de blocage 140est maintenue dans sa position fléchie et l'opérateur peut lâcher le dispositif

Lorsqu'il veut libérer la sonde, l'opérateur exerce une force sur cette languette 123 à l'opposé de la pièce de blocage 140, si bien que la dent 143 se libère des crans de la face distale 122 et, par élasticité, la pièce de blocage 140 revient à sa position d'équilibre où elle est éloignée de l'axe X-X' et donc de la sonde.

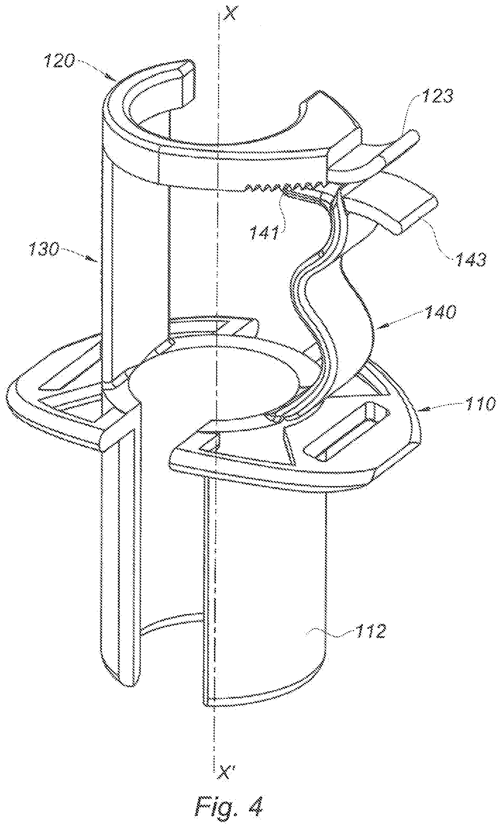

Dans la variante illustrée en figure 4, la pièce de blocage 140 comporte également une languette 143 à son extrémité proximale, qui s'étend à l'opposé de l'excroissance 141. Les deux languettes 123 et 143 sont courbes et s'étendent radialement en s'écartant l'une de l'autre afin de faciliter l'insertion de la sonde entre la pièce de blocage 140 et t'anneau 120.

Il est important de noter que le dispositif n'est pas prévu, comme certains dispositifs de l'état de l'art, pour interrompre la circulation d'une fluide liquide ou gazeux dans la sonde. Il est même intrinsèquement prévu pour qu'une telle interruption, qui pourrait avoir, à la suite d'une erreur de manipulation, des conséquences gravissimes, soit impossible. En effet, si l'opérateur exerçait un trop grand effort de pincement qui aboutirait à ce que la sonde se trouve écrasée entre l'excroissance 141 et la face interne 131 de la pièce d'écartement, la pièce de blocage 140 une fois relâchée reviendrait en prise avec les crans de la face distale 122.

Il ressort de la description qui précède que le dispositif est monobloc, ce qui permet de le mettre en place d'une seule main. De plus, ceci évite les risques de dissociation et de perte de pièce. Le dispositif peut être réalisé par moulage par injection.

Cette description n'est pas limitative. Ainsi :
- La pièce d'appui pourrait prendre une autre forme, par exemple circulaire, polygonale, voire prendre la forme de branches rayonnant autour de l'axe X-X',
- L'anneau du moyen de guidage pourrais prendre une forme autre que circulaire, par exemple ovale ou polygonale,
- La pièce d'écartement 130 pourrait être plane,
- la pièce de blocage 140 pourrait ne pas être ondulée, notamment être rectiligne ou simplement courbe,
- l'excroissance 141 pourrait présenter une autre forme que celle d'un bec ; elle pourrait présenter une ferme de patin, augmentant ainsi l'effet de frottement contre la sonde.

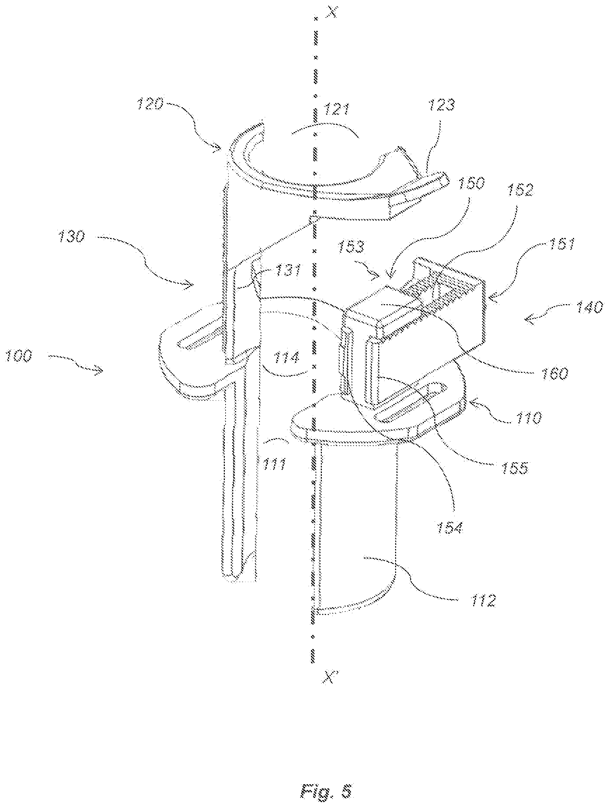

Tel qu'illustré sur la figure 5, un exemple de réalisation selon l'invention comprend un moyen de blocage 140, ledit moyen de blocage comportant un plot 150 disposé sur le moyen d'appui 110 et s'étendant le long de l'axe X-X' à partir de la face proximale dudit moyen d'appui, diamétralement opposée à la face interne 131 de la pièce d'écartement 130. Le moyen de blocage 140 peut, en outre, comporter un fermoir 151 destiné à coulisser le long du plot 150.

Selon un exemple de réalisation de l'invention, le fermoir 151 présente la forme d'un prisme droit, par exemple un parallélépipède rectangle. Le fermoir 151 comprend dans une partie centrale de son volume une tranchée 152 de largeur donnée, s'étendant suivant un axe longitudinal dudit fermoir et formant deux bras longitudinaux. Lesdits deux bras longitudinaux sont destinés à s'insérer autour des faces latérales du plot 150. En particulier, lesdits deux bras longitudinaux sont prévus pour coopérer avec les faces du plot 151 de manière à guider le fermoir 150 pour sa mise en place le long du moyen d'appui 110 jusqu'à une position finale de fixation, dans laquelle le fermoir devient solidaire dudit moyen d'appui, et du dispositif 100.

Lorsque le fermoir 151 est coulissé, par exemple partiellement, le long du plot 150, le moyen de blocage 140 permet de bloquer une sonde dont les deux extrémités sont engagées, pour maintenir ladite sonde dans le dispositif 100. Compte tenu de sa position diamétralement opposée par rapport à la pièce d'écartement 130, on comprend que lorsque le fermoir 151 est coulissé le long du plot 150, ladite sonde est bloquée par frottement entre la face interne 131 de la pièce d'écartement 130 et une face latérale interne 153 du fermoir 150,

Selon un exemple de réalisation de l'invention, le fermoir 151 comporte un prisme droit à base triangulaire. Selon cet exemple, le coulissement du fermoir 151 le long du plot 150 permet d'exercer une pression latérale progressive sur une sonde insérée dans le dispositif 100 le long de l'axe X -X', en fonction de la longueur dudit fermoir qui est coulissée le long dudit plot. En effet, un fermoir 151 comprenant un prisme droit à base triangulaire permet de modifier progressivement la taille de l'ouverture axiale 114 du dispositif. 100 lorsque ledit fermoir est coulissé le long du plot 150. La qualité du blocage d'une sonde dans le dispositif. 100 peut ainsi être adaptée en fonction du diamètre de la sonde insérée dans ledit dispositif. En particulier, le blocage d'une sonde de petit diamètre ou de grand diamètre peut être assuré par un coulissement plus ou moins important du fermoir 150 le long du plot 150.

Selon un exemple de réalisation de l'invention, la base supérieure du plot 150 comporte en outre une face qui s'étend le long du moyen d'appui 110 et dont la largeur est supérieure à l'écartement des bras longitudinaux de la tranchée 152 du fermoir 151. En particulier, le plot 150 est un bloc en forme de « I », dont la partie centrale est destinée à coopérer avec le fermoir 151. Ceci permet d'assurer le blocage du fermoir 151 dans une direction parallèle à l'axe X-X' lorsque ledit fermoir est coulissé au moins partiellement le long du plot. Sous l'application d'une pression suffisante exercée par l'utilisateur, le fermoir 151 peut aussi être déplacé selon un mouvement de retour pour libérer une sonde engagée dans le dispositif 100. Avantageusement, l'opérateur peut, avec un ou deux doigts d'une même main, appuyer sur le fermoir 151 pour le faire coulisser longitudinalement le long du plot 151. Lorsqu'il veut libérer la sonde, l'opérateur exerce une force sur le fermoir 151 pour le faire coulisser et libérer les crans des faces latérales du plot 150. Le moyen de blocage 140 est ainsi éloigné de l'axe X-X' et donc de la sonde. Pour assurer un meilleur blocage de la sonde, au moins l'une des faces du fermoir 151 et/ou du plot 150 peut être traitée de façon à posséder une propriété antidérapante.

Selon un exemple de réalisation de l'invention, le fermoir 151 comporte en outre des moyens d'anti-retour. Selon cet exemple de réalisation, ces moyens d'anti-retour comprennent des crans disposés dans la tranchée 152 du fermoir 151, et agencés pour s'accrocher à un bord saillant formé à la périphérie du plot 150. Avantageusement, ces crans permettent une meilleure fixation du fermoir 151 lorsque celui-ci est coulissé sur une longueur prédéterminée d'une surface latérale dudit plot. A cet effet, les deux faces internes de la tranchée 142 dudit fermoir comportent chacune au moins un cran prévu pour coopérer avec l'une des faces du plot 151, de façon à former un moyen d'anti-retour. Avantageusement, les crans sont disposés l'un en face de l'autre dans la tranchée 152. La coopération de ces crans avec les faces latérales du plot 150 permettent d'empêcher un mouvement longitudinal de retour du fermoir.

## Revendications

1. Dispositif (100) de maintien d'une sonde de mise en communication de l'intérieur du corps d'un patient avec l'extérieur, comprenant, disposés le long d'un axe longitudinal (X-X') du dispositif :
- à son extrémité distale, un moyen d'appui (110) du dispositif sur le corps du patient qui s'étend radialement, comportant une ouverture axiale (114) pour le passage de ladite sonde et comportant une première ouverture radiale (111) communiquant avec ladite ouverture axiale,
- à son extrémité proximale, un moyen de guidage (120) de ladite sonde comportant une seconde ouverture radiale (121),
- un moyen d'écartement longitudinal (130) dudit moyen d'appui et dudit moyen de guidage,
et **caractérisé en ce que** ladite première ouverture radiale (111) et la seconde ouverture radiale (121) sont sensiblement diamétralement opposées par rapport à l'axe longitudinal (X-X') du dispositif.

2. Dispositif de maintien d'une sonde selon l'une des revendications précédentes, **caractérisé en ce que** le moyen d'appui (110) est prolongé sur sa face distale par un corps cylindrique (112) coaxial comportant une fente longitudinale (113), ladite fente étant en communication avec la première ouverture radiale (111) du moyen d'appui.

3. Dispositif de maintien d'une sonde selon l'une des revendications précédentes, **caractérisé en ce que** le moyen de guidage (120) comporte une excroissance (124) qui s'étend radialement en direction opposée à la pièce d'écartement (130).

4. Dispositif de maintien d'une sonde selon la revendication 3, **caractérisé en ce que** le moyen de guidage (120) comporte une languette (123) qui s'étend radialement à partir de l'excroissance (124).

5. Dispositif de maintien d'une sonde selon l'une des revendications précédentes, **caractérisé en ce qu'**il comporte en outre un moyen de blocage (140) prévu pour bloquer la sonde contre le moyen d'écartement (130) lorsque ladite sonde s'étend selon l'axe longitudinal (X-X') du dispositif.

6. Dispositif de maintien d'une sonde selon la revendication 5, **caractérisé en ce que** le moyen de blocage comprend une lame (140) qui s'étend longitudinalement à partir de la face proximale (110a) du moyen d'appui (110), diamétralement opposée au moyen d'écartement (130).

7. Dispositif de maintien d'une sonde selon la revendication 6, **caractérisé en ce que** la lame (140) présente une forme radialement incurvée et comporte une excroissance (141) qui s'étend vers l'axe longitudinal (X-X') du dispositif, de façon à bloquer la sonde entre ladite partie de blocage et le moyen d'écartement (130) lorsqu'on exerce une force sur ladite lame.

8. Dispositif de maintien d'une sonde selon l'une des revendications 5 à 7, **caractérisé en ce que** la face interne du moyen d'écartement (130) est conformée ou traitée de façon à posséder une propriété antidérapante.

9. Dispositif de maintien d'une sonde selon l'une des revendications 5 à 8, **caractérisé en ce qu'**il comporte en outre des moyens d'anti-retour du moyen de blocage (140) dans la position où l'excroissance (141) est rapprochée du moyen d'écartement (130).

10. Dispositif de maintien d'une sonde selon la revendication 9, caractérisé et en ce que les moyens d'anti-retour comportent une surface crantée (122) sur une face distale de l'excroissance (124), lesdits crans étant prévus pour coopérer avec une dent (143) située à l'extrémité proximale du moyen de blocage (140).

11. Dispositif de maintien d'une sonde selon l'une des revendications précédentes, **caractérisé en ce qu'**il est monobloc.

12. Dispositif de maintien d'une sonde selon la revendication 5, **caractérisé en ce que** ce que le moyen de blocage (140) comporte un plot (150) disposé sur le moyen d'appui (110) et s'étendant le long de l'axe longitudinal (X-X') du dispositif à partir de la face proximale dudit moyen d'appui, diamétralement opposée à la face interne (131) de la pièce d'écartement (130).

13. Dispositif de maintien d'une sonde selon l'une des revendications précédentes, **caractérisé en ce qu'**il est réalisé en une matière plastique à usage pharmaceutique ou médico-chirurgical

14. Procédé de mise en place d'un dispositif (100) de maintien d'une sonde selon l'une des revendications précédentes, ladite sonde étant déjà en place sur le corps du patient, comportant les étapes consistant à :
- Introduire la sonde entre l'extrémité proximale du moyen de blocage (140) et le moyen de guidage (120), puis soit:
- Faire pénétrer la sonde dans la première ouverture radiale (111) du moyen d'appui (100), et
- Faire pénétrer la sonde dans la seconde ouverture radiale du moyen guidage ou alternativement:
- Faire pénétrer la sonde dans la seconde ouverture radiale (121) du moyen de guidage (120), et
- Faire pénétrer la sonde dans la première ouverture radiale du moyen d'appui;
de façon à ce que la sonde s'étende selon l'axe longitudinal (X-X') du dispositif.

## Patentansprüche

1. Vorrichtung (100) zum Halten einer Kommunikationsherstellungssonde von dem Inneren des Körpers eines Patienten nach außen, die, entlang einer Längsachse (X-X') der Vorrichtung angeordnet, Folgendes umfasst:
- an ihrem distalen Ende ein Auflagemittel (110) der Vorrichtung auf dem Körper des Patienten, das sich radial erstreckt, eine axiale Öffnung (114) für die Passage der Sonde einschließt, und eine erste radiale Öffnung (111), die mit der axialen Öffnung in Kommunikation steht, einschließt,
- an ihrem proximalen Ende ein Führungsmittel (120) der Sonde, das eine zweite radiale Öffnung (121) einschließt,
- ein Mittel zum Längsabspreizen (130) des Auflagemittels und des Führungsmittels,
und **dadurch gekennzeichnet, dass** die erste radiale Öffnung (111) und die zweite radiale Öffnung (121) im Wesentlichen bezüglich der Längsachse (X-X') der Vorrichtung diametral entgegengesetzt sind.

2. Vorrichtung zum Halten einer Sonde nach einem der vorstehenden Ansprüche, **dadurch gekennzeichnet, dass** das Auflagemittel (110) auf seiner distalen Seite durch einen koaxialen zylindrischen Körper (112) verlängert ist, der einen Längsschlitz (113) einschließt, wobei der Schlitz mit der ersten radialen Öffnung (111) des Auflagemittels in Kommunikation steht.

3. Vorrichtung zum Halten einer Sonde nach einem der vorstehenden Ansprüche, **dadurch gekennzeichnet, dass** das Führungsmittel (120) eine Ausstülpung (124) einschließt, die sich radial in entgegengesetzte Richtung zu dem Abspreizteil (130) erstreckt.

4. Vorrichtung zum Halten einer Sonde nach Anspruch 3, **dadurch gekennzeichnet, dass** das Führungsmittel (120) eine Lasche (123) einschließt, die sich radial ausgehend von der Ausstülpung (124) erstreckt.

5. Vorrichtung zum Halten einer Sonde nach einem der vorstehenden Ansprüche, **dadurch gekennzeichnet, dass** sie weiter ein Blockierungsmittel (140) einschließt, das vorgesehen ist, um die Sonde gegen das Abspreizmittel (130) zu blockieren, wenn sich die Sonde entlang der Längsachse (X-X') der Vorrichtung erstreckt.

6. Vorrichtung zum Halten einer Sonde nach Anspruch 5, **dadurch gekennzeichnet, dass** das Blockierungsmittel eine Klinge (140) umfasst, die sich längs ausgehend von der proximalen Seite (110a) des Auflagemittels (110), dem Abspreizmittel (130) diametral entgegengesetzt erstreckt.

7. Vorrichtung zum Halten einer Sonde nach Anspruch 6, **dadurch gekennzeichnet, dass** die Klinge (140) eine radial gebogene Form aufweist und eine Ausstülpung (141) einschließt, die sich zu der Längsachse (X-X') der Vorrichtung derart erstreckt, dass die Sonde zwischen dem Blockierungsteil und dem Abspreizmittel (130) blockiert wird, wenn auf der Klinge eine Kraft ausgeübt wird.

8. Vorrichtung zum Halten einer Sonde nach einem der Ansprüche 5 bis 7, **dadurch gekennzeichnet, dass** die innere Seite des Abspreizmittels (130) derart ausgestaltet oder behandelt ist, dass sie eine Rutschfestigkeitseigenschaft besitzt.

9. Vorrichtung zum Halten einer Sonde nach einem der Ansprüche 5 bis 8, **dadurch gekennzeichnet, dass** sie weiter Rückkehrschutzmittel des Blockierungsmittels (140) in die Position, in der die Ausstülpung (141) dem Abspreizmittel (130) angenähert ist, einschließt.

10. Vorrichtung zum Halten einer Sonde nach Anspruch 9, **dadurch gekennzeichnet, dass** die Rückkehrschutzmittel eine Rastkerbenoberfläche (122) auf einer distalen Seite der Ausstülpung (124) einschließen, wobei die Rastkerben vorgesehen sind, um mit einem Zahn (143), der an dem proximalen Ende des Blockierungsmittels (140) liegt, zusammenzuwirken.

11. Vorrichtung zum Halten einer Sonde nach einem der vorstehenden Ansprüche, **dadurch gekennzeichnet, dass** sie einstückig ist.

12. Vorrichtung zum Halten einer Sonde nach Anspruch 5, **dadurch gekennzeichnet, dass** das Blockierungsmittel (140) einen Klotz (150) einschließt, der auf dem Auflagemittel (110) angeordnet ist und sich entlang der Längsachse (X-X') der Vorrichtung ausgehend von der proximalen Seite des Auflagemittels, der inneren Seite (131) des Abspreizteils (130) entgegengesetzt, erstreckt.

13. Vorrichtung zum Halten einer Sonde nach einem der vorstehenden Ansprüche, **dadurch gekennzeichnet, dass** sie aus einem Kunststoffmaterial für pharmazeutische oder medizinisch-chirurgische Zwecke hergestellt ist.

14. Verfahren zum Umsetzen einer Vorrichtung (100) zum Halten einer Sonde nach einem der vorstehenden Ansprüche, wobei die Sonde an dem Körper des Patienten bereits angelegt ist, das die Schritte einschließt, die bestehen aus:
- Einführen der Sonde zwischen dem proximalen Ende des Blockierungsmittels (140) und dem Führungsmittel (120), dann entweder:
- Eindringen lassen der Sonde in die erste radiale Öffnung (111) des Auflagemittels (100), und
- Eindringen lassen der Sonde in die zweite radiale Öffnung des Führungsmittels oder alternativ:
- Eindringen lassen der Sonde in die zweite radiale Öffnung (121) des Führungsmittels (120), und
- Eindringen lassen der Sonde in die erste radiale Öffnung des Auflagemittels;
so dass sich die Sonde entlang der Längsachse (X-X') der Vorrichtung erstreckt.

## Claims

1. Device (100) for holding a probe for putting the inside of the body of patient in communication with the outside, comprising, arranged along a longitudinal axis (X-X') of the device:
- at the distal end thereof, means (110) for supporting the device on the body of the patient that extends radially, comprising an axial opening (114) for passage of said probe and comprising a first radial opening (111) communicating with said axial opening,
- at the proximal end thereof, means (120) for guiding said probe comprising a second radial opening (121),
- a means (130) for the longitudinal separation of said supporting means and said guidance means,
and **characterised in that** said first radial opening (111) and the second radial opening (121) are substantially diametrically opposed with respect to the longitudinal axis (X-X') of the device.

2. Device for holding a probe according to one of the preceding claims, **characterised in that** the supporting means (110) is extended on the distal face thereof by a coaxial cylindrical body (112) comprising a longitudinal slot (113), said slot being in communication with the first radial opening (111) of the support means.

3. Device for holding a probe according to one of the preceding claims, **characterised in that** the guide means (120) comprises a protrusion (124) that extends radially in the opposite direction to the separation piece (130).

4. Device for holding a probe according to claim 3, **characterised in that** the guide means (120) comprises a tongue (123) that extends radially from the protrusion (124).

5. Device for holding a probe according to any of the preceding claims, **characterised in that** it further comprises a locking means (140) provided for locking the probe against the separation means (130) when said probe extends along the longitudinal axis (X-X') of the device.

6. Device for holding a probe according to claim 5, **characterised in that** the locking means comprises a blade (140) that extends longitudinally from the proximal face (110a) of the support means (110), diametrically opposed to the separation means (130).

7. Device for holding a probe according to claim 6, **characterised in that** the blade (140) has a radially curved shape and comprises a protrusion (141) that extends towards the longitudinal axis (X-X') of the device, so as to lock the probe between said locking part and the separation means (130) when a force is exerted on said blade.

8. Device for holding a probe according to one of claims 5 to 7, **characterised in that** the internal face of the separation means (130) is shaped or treated so as to have a nonslip property.

9. Device for holding a probe according to one of claims 5 to 8, **characterised in that** it further comprises means for non-return of the locking means (140) in the position where the protrusion (141) is close to the separation means (130).

10. Device for holding a probe according to claim 9, **characterised in that** the non-return means comprises a notched surface (122) on a distal face of the protrusion (124), said notches being provided to cooperate with a tooth (143) situated at the proximal end of the locking means (140).

11. Device for holding a probe according to one of the preceding claims, **characterised in that** it is in a single piece.

12. Device for holding a probe according to claim 5, **characterised in that** the locking means (140) comprises a stud (150) arranged on the support means (110) and extending along the longitudinal axis (X-X') of the device from the proximal face of said support means, diametrically opposed to the internal face (131) of the separation piece (130).

13. Device for holding a probe according to one of the preceding claims, **characterised in that** it is produced from a plastic material for pharmaceutical or medicosurgical use.

14. Method for positioning a device (100) for holding a probe according to one of the preceding claims, said probe already being in place on the body of the patient, comprising steps consisting of:
- introducing the probe between the proximal end of the locking means (140) and the guidance means (120), and then either:
- causing the probe to enter the first radial opening (111) of the support means (100), and
- causing the probe to enter the second radial opening of the guide means, or alternatively:
- causing the probe to enter the second radial opening (121) of the guide means (120), and
- causing the probe to enter the first radial opening of the support means;
such that the probe extends along the longitudinal axis (X-X') of the device.
